# EUROPEAN PATENT SPECIFICATION

(11) **EP 0 276 964 B1**
(45) Date of publication and mention of the grant of the patent: **24.03.1993**
(21) Application number: 88300578.7
(22) Date of filing: 25.01.1988
(51) Int. Cl.: A61B 17/06

(54) **A package containing a suture**
Packung für Nahtmaterial
Assemblage contenant une suture

(30) Priority: 26.01.1987 JP 9671/87 U
(43) Date of publication of application: 03.08.1988
(73) Proprietor: NIPPON SHOJI KABUSHIKI KAISHA, Osaka 540 (JP)
(72) Inventor: Okuhara, Makoto, Higashikatsushikagun Chiba (JP); Ishida, Yasumi, Satsuteshi Saitama (JP)
(74) Representative: Cook, Anthony John

(56) References cited:
- EP-A- 0 086 657
- EP-A- 0 206 698
- FR-A- 2 390 938
- US-A- 4 249 656
- US-A- 4 483 437

## Description

The present invention relates to a package containing a suture or surgical thread. More particularly, the present invention relates to a package containing a suture in such a manner that the suture has no sharply bent portion or curling tendency when it is unpacked for use, the suture either not being provided with a needle or having a needle at one or both ends. In this specification the former is referred to as an unneedled suture and the latter as a needled suture which may be single needled or double needled. However, unless specified to the contrary the term "suture" includes unneedled and needled sutures.

There are at least three kinds of sutures; one is unneedled, an other is single-needled, and a third is double-needled. When a suture is to be placed in a package the common practice is that after the suture has been cut to a predetermined length, it is wound around a plurality of pins on the package material and the package is then closed. The package containing the suture is wrapped in a plastics film, an aluminium foil or a composite sheet so as to protect the packed suture against moisture and dirt. When the packed suture is to be removed for use, the package is unwrapped, and the end of the suture or the needle attached thereto is picked up through an opening in the package.

In packing a suture there are two important points to be observed; the first is to accommodate the suture in the package with no tangle or sharply bent portion, and the second is to design the package in such a manner as to enable it to be readily opened and allow the user to pick up the packed suture smoothly.

To meet these requirements many proposals have been made, among which are those disclosed in Japanese Patent Publications (unexamined) Nos 58(1983)-149750, 59(1984)-228845 and 60(1985)-148550.

The prior art packages consist of panels folded at least into three parts, so as to have three functionally distinguished sections (EP-A-0 086 657, patent family member of JP-A-58-149 750). For example, a single-needled suture is accommodated in such a manner that the wound suture and its unneedled end are located in one section and the needle in another. The package disclosed in the No 60-148550 Specification has five panels connected to each other by folds. The panels consist of a suture-retaining panel, a needle-retaining panel, a needle-covering panel, a suture-covering panel and a side panel. When the suture is provided with a needle at an end, the main body of the suture is wound around pins which protrude through four apertures produced in the suture-retaining panel, and the needle and the part of suture adjacent to the needle are inserted in respective slits produced in the needle-retaining panel. The suture-covering panel is folded onto the suture-retaining panel, and the needle-covering panel is folded onto the suture-covering panel. Finally, the side panel is folded onto the needle-covering panel. In this way a finished package is produced.

The provision of the functionally distinguished panels is advantageous in that the suture is prevented from becoming tangled, and that it is convenient for the user when the suture is unpacked from the package. However it is labour-consuming to provide as many as five folded panels, and care must be especially taken when the needle is removed through the slit in the package. Another disadvantage is that the part of suture adjacent to the needle becomes bent or curls.

According to the present invention there is provided a suture-containing package comprising:
a substrate having a first panel and a second panel thermally sealed together along at least two edges thereof, thereby forming a suture compartment therebetween, and at at least two spot locations;
spaced pairs of apertures or single apertures provided in regions of the panels forming the suture compartment, the apertures enabling removable pins about which a suture can be wound to pass through the package;
the first panel including a tear line extending along a first edge of the suture compartment so that the first panel can be readily torn along the tear line;
the second panel having an inclined edge beyond which a portion of the first panel protrudes to form a tab so that the user can grasp the tab and tear the first panel along the tear line; and said suture compartment accommodating a main portion of said suture whose end portions are located outside the compartment between said spot locations.

Some embodiments of the invention will now be described, by way of examples, with reference to the accompanying drawings, in which:-
Figure 1 is a front view showing a package embodying the present invention, the package being wrapped in a sealing film;
Figure 2 is a front view showing the package of Figure 1 when an openable section is opened;
Figure 3 is a front view showing the package of Figure 1 in its pre-folded state;
Figure 4 is a front view showing a modified version of the package;
Figures 5(a) and 5(b) are front views showing the first panel and the second panel, respectively of the modified form of package; and
Figure 6 is a front view showing a package storing a suture having no needle.

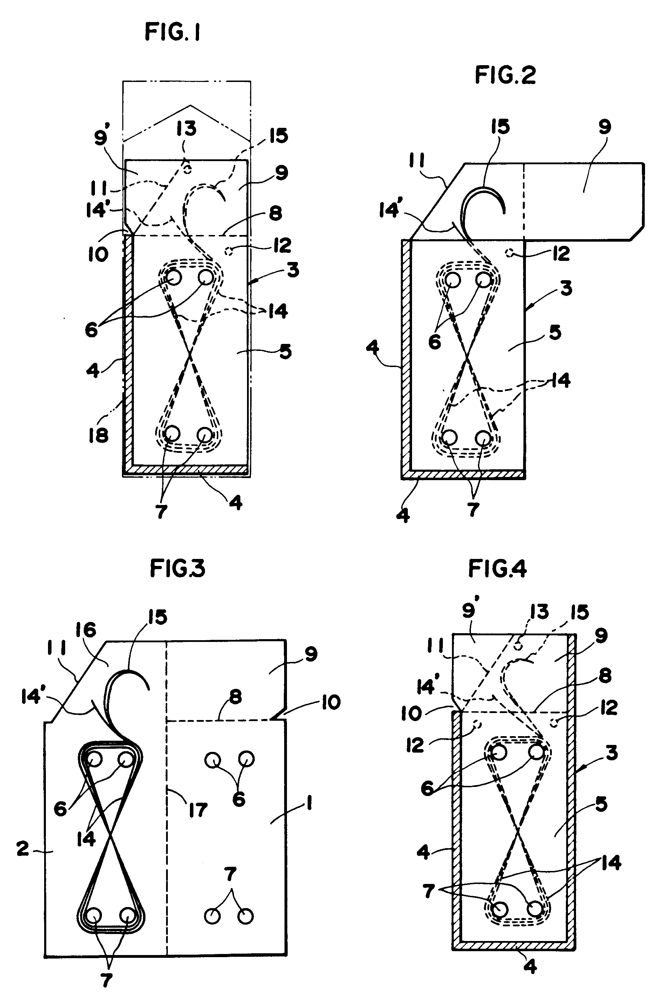

The package shown in Figures 1 to 3 has a first (front) panel 1 and a second (rear) panel 2 foldable along a longitudinal folding line 17, (hereinafter referred to as "fold"). The two panels 1 and 2 are made of thick paper and coated with polyethylene on their sides which form inner surfaces when folded. The panels 1 and 2 are preferably rectangular in shape. When they are folded about line 17 and overlaid one on the other, their two unconnected side edges which intersect at a right angle are thermally bonded together to form a substrate 3. In the illustrated embodiment, the unconnected longitudinal edges and the lower edges are bonded together by a heat seal 4. There is formed a space between the panels 1 and 2, and this space provides a compartment 5 which accommodates a suture 14. The panels forming the suture compartment 5 are provided with two pairs of apertures 6 and 7, vertically spaced from each other, through which removable pins (not shown) are inserted for winding the suture 14 around. It is possible to reduce the number of the apertures 6 and 7 in each panel to two, one above the other. However, it is preferred to have one pair above the other pair to give a total of four apertures in each panel, which prevents the suture 14 from becoming tangled or twisted. Actually the number of apertures depends on the size of the package, and the thickness and length of the suture 14. The shapes of the apertures 6 and 7 are normally circular, but they can be vertically elongated so that the spacing between the pins may be adjusted to wind different lengths of sutures around the pins. The suture compartment 5 is provided with a line 8, along which an opener section 9 of the first panel 1 is torn as shown in Figure 2. The line 8 has a tear notch at one end for facilitating the tearing of the opener section 9 along the line 8. The line 8 may be weakened or perforated or comprise a stitch. The second panel 2 has an inclined edge 11 extending diagonally from the tear notch 10. The reference number 9ʹ denotes a tab portion of section 9 which projects over and beyond the edge 11 of the second panel 2. The user grasps the tab 9ʹ and tears the opener section 9 along the line 8. The two panels 1 and 2 are additionally bonded together by heat at spots 12 and 13, which are located one above the other to the accommodated suture end portions including the needle or needles 15. Thus, the suture ends are secured in the package.

The suture 14 is accommodated in the suture compartment 5, wherein it is wound in the form of numeral "8" around the pins inserted through the apertures 6 and 7. The needle 15 and the end 14ʹ of the suture 14 are located outside the suture compartment 5, that is, in an upper space between the panels 1 and 2.

The suture 14 is accommodated in the following manner:
Referring to Figure 3, pins (not shown) are inserted through the apertures 6 and 7 in the second panel 2. The suture 14 is folded in half, or when required, in more folds; in the illustrated embodiment it is provided with a needle 15 at one end, and the other end 14ʹ has no needle. Then it is wound around the pins in the form of numeral "8", wherein the terminal end 14ʹ and the needle 15 are located on the upper side 16 of the second panel 2 as best shown in Figure 3. Then, as shown in Figure 1 the first panel 1 is folded along the fold line 17 over the second panel 2 in such a manner that the pins extend through the apertures 6 and 7 in the first panel 1. Finally, the panels 1 and 2 are thermally bonded together at 4, 12 and 13. The package is then released from the pins.

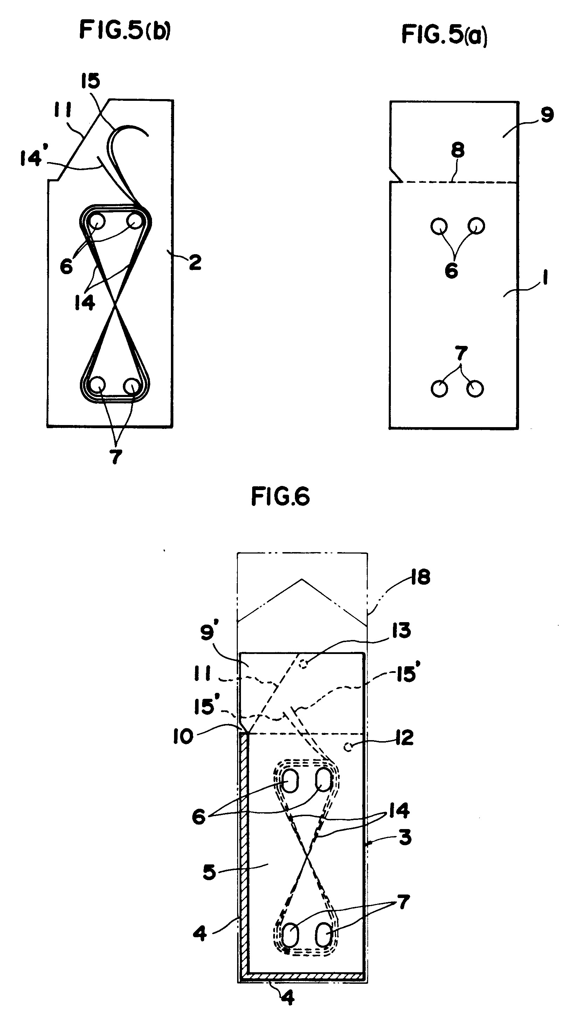

Figures 5(a) and 5(b) show a modified version of the package, in which the first panel 1 and the second panel 2 are initially separate from each other. They are then thermally bonded together along side and bottom edges and by heat spots in the same manner as mentioned above, and the finished package is as shown in Figure 4. Figure 6 shows an example in which the suture 14 has no needle, and both ends 15ʹ of the suture 14 are located in an upper space between the panels 1 and 2.

The thermally sealable panels can be made of relatively stiff plastics sheet, such as polyethylene sheet, and it is possible to coat the inner forming sides of the panels with polyethylene or polyproylene. The package is elongate as shown in the drawings, and the dimensional ratio of the overall length of the package to that of the suture compartment is in the range of 3:2 to 4:3.

The suture 14 is safely accommodated in the package without becoming tangled, sharply bent and/or curled, and also it is protected against moisture and dirt. When the suture 14 is to be unpacked the user has only to grip the tab 9ʹ and tear the opener section 9 along the line 8. Then the user picks up the needle 15 and the end 14ʹ or both ends 15ʹ. The suture 14 has no tendency to bend or curl when it is used in a surgical operation.

## Claims

1. A suture-containing package comprising:
a substrate having a first panel (1) and a second panel (2) thermally sealed together along at least two edges thereof, thereby forming a suture compartment (5) therebetween, and at at least two spot locations (12, 13);
spaced pairs of apertures (6, 7) or single apertures provided in regions of the panels (1 and 2) forming the suture compartment (5), the apertures (6, 7) enabling removable pins about which a suture (14) can be wound to pass through the package;
the first panel (1) including a tear line (8) extending along a first edge of the suture compartment (5) so that the first panel (1) can be readily torn along the tear line (8);
the second panel (2) having an inclined edge (11) beyond which a portion (9) of the first panel (1) protrudes to form a tab (9ʹ) so that the user can grasp the tab (9ʹ) and tear the first panel (1) along the tear line (8); and said suture compartment (5) accommodating a main portion of said suture (14) whose end portions are located outside the compartment (5) between said spot locations (12, 13).

2. A suture-containing package as claimed in claim 1, in which the apertures (6, 7) are in pairs in each of the upper part and the lower part of the suture compartment (5).

3. A suture-containing package as claimed in claim 1 or claim 2, in which the suture (14) is provided with a needle at one or both ends.

4. A suture-containing package as claimed in any preceding claim, in which the substrate comprises a single sheet which is foldable into said first and second panels (1 and 2).

5. A suture-containing package as claimed in any preceding claim, in which the main portion of the suture (14) is accommodated in the shape of numeral "8".

6. A suture-containing package as claimed in any preceding claim, in which the ratio of the length of the compartment (5) to the length of the whole package is in the range of 2:3 to 3:4.

## Patentansprüche

1. Packung für Nahtmaterial, die
ein Substrat, das ein erstes Feld (1) und ein zweites Feld (2) hat, welche mindestens entlang zweier ihrer Kanten (wodurch zwischen ihnen ein Fach für Nahtmaterial (5) gebildet wird) und an mindestens zwei Punktstellen (12, 13) heißesiegelt sind,
mit Zwischenräumen angeordnete Öffnungspaare (6, 7) oder einzelne Öffnungen, die in das Fach für Nahtmaterial (5) bildenden Felderbereichen (1 und 2) vorgesehen sind, wobei die Öffnungen (6, 7) entfernbare Stifte ermöglichen, um welche ein Nahtmaterial (14) für das Ziehen durch die Packung herumgewickelt werden kann, umfaßt,
wobei das erste Feld (1) eine sich entlang einer ersten Kante des Faches für Nahtmaterial (5) erstreckende Reißlinie (8) einschließt, so daß das erste Feld (1) leicht entlang der Reißlinie (8) gerissen werden kann, und
das zweite Feld (2) eine schräge Kante (11) hat, über welche ein Teilstück (9) des ersten Feldes (1) hinausragt, um einen Streifen (9') zu bilden, so dar der Verwender den Streifen (9') erfassen und das erste Feld (1) entlang der Reißlinie (8) reissen kann, wobei das Fach für das Nahtmaterial (5) Platz für einen Großteil des Nahtmaterials (14), dessen Endabschnitte sich außerhalb des Fachs (5) zwischen den Punktstellen (12, 13) befinden, bietet.

2. Packung für Nahtmaterial nach Anspruch 1, bei welcher sich die Öffnungen (6,7) jeweils in Paaren im oberen Teil und im unteren Teil des Faches für Nahtmaterial (5) befinden.

3. Packung für Nahtmaterial nach Anspruch 1 oder Anspruch 2, bei welcher das Nahtmaterial (14) mit einer Nadel an einem oder beiden Enden versehen ist.

4. Packung für Nahtmaterial nach einem der vorhergehenden Ansprüche, bei welcher das Substrat eine einzelne Folie umfaßt, die zu den ersten und zweiten Feldern (1 und 2) zu falten ist.

5. Packung für Nahtmaterial nach einem der vorhergehenden Ansprüche, in welcher der Großteil des Nahtmaterials (14) in Form der Zahlennummer "8" untergebracht ist.

6. Packung für Nahtmaterial nach einem der vorhergehenden Ansprüche, bei welcher das Verhältnis der Länge des Faches (5) zu der Länge der ganzen Packung in dem Bereich von 2:3 bis 3:4 liegt.

## Revendications

1. Emballage contenant un fil de suture comprenant :
un support comportant un premier panneau (1) et un second panneau (2) thermosoudés l'un à l'autre le long d'au moins deux de leurs bords, de manière à former un compartiment (5) de fil entre eux, et en au moins deux points (12, 13);
des paires espacées d'ouvertures (6, 7) ou des ouvertures simples situées dans des zones des panneaux (1 et 2) formant le compartiment (5) de fil de suture, les ouvertures (6, 7) permettant à des tiges amovibles autour desquelles un fil de suture (14) peut être enroulé de traverser l'emballage;
le premier panneau (1) comprenant une ligne de déchirage (8) s'étendant le long d'un premier bord du compartiment (5) de fil de manière que le premier panneau (1) puisse être facilement déchiré le long de la ligne de déchirage (8);
le second panneau (2) comportant un bord incliné (11) au-delà duquel une portion (9) du premier panneau (1) se projette pour former une languette (9') de manière que l'utilisateur puisse saisir la languette (9') et déchirer le premier panneau (1) le long de la ligne de déchirage (8); et ledit compartiment (5) de fil de suture logeant une portion principale dudit fil de suture (14) dont les portions d'extrémité sont situées hors du compartiment (5) entre lesdits points (12, 13).

2. Emballage contenant un fil de suture selon la revendication 1, dans lequel les ouvertures (6, 7) sont en paires dans chaque partie supérieure et partie inférieure du compartiment (5) de fil de suture.

3. Emballage contenant un fil de suture selon la revendication 1 ou 2, dans lequel le fil de suture (14) est doté d'une aiguille à une extrémité ou aux deux extrémités.

4. Emballage contenant un fil de suture selon l'une quelconque des revendications précédentes, dans lequel le support comprend une seule feuille qui peut être pliée en lesdits premier panneau (1) et second panneau (2).

5. Emballage contenant un fil de suture selon l'une quelconque des revendications précédentes, dans lequel la portion principale du fil de suture (14) est logée en un "8".

6. Emballage contenant un fil de suture selon l'une quelconque des revendications précédentes, dans lequel le rapport de la longueur du compartiment (5) à celle de l'emballage tout entier est compris entre 2:3 et 3:4.
